# EUROPEAN PATENT SPECIFICATION

(11) **EP 2 782 605 B1**
(45) Date of publication and mention of the grant of the patent: **29.03.2017**
(21) Application number: 12787029.3
(22) Date of filing: 19.11.2012
(51) Int. Cl.: A61L 9/12

(54) **FRAGRANCE DIFFUSER, PARTICULARLY FOR SMALL SPACES, AND ASSOCIATED PRODUCTION PROCESS**
DUFTSTOFFDIFFUSOR, INSBESONDERE FÜR KLEINE RÄUME, UND ENTSPRECHENDES HERSTELLUNGSVERFAHREN
DIFFUSEUR DE PARFUM, NOTAMMENT POUR PETITS ESPACES, ET PROCÉDÉ DE PRODUCTION ASSOCIÉ

(30) Priority: 23.11.2011 IT MO20110301
(43) Date of publication of application: 01.10.2014
(73) Proprietor: FD Packaging Società a Responsabilità Limitata Semplificata, 41030 San Prospero (MO) (IT)
(72) Inventor: FREDDI, Antonio, I-42046 Reggiolo (IT); FREDDI, Diego, I-42046 Reggiolo (IT); DALLASTA, Emanuele, I-42016 Guastalla (IT); TABARELLI, Primo, I-46029 Suzzara (IT)
(74) Representative: Modiano, Micaela Nadia
(86) International application number: PCT/EP2012/072967
(87) International publication number: WO 2013/076033

(56) References cited:
- WO-A1-2009/033509
- DE-U1- 20 114 352
- US-A- 4 998 671
- US-A1- 2011 180 621

## Description

The present invention relates to a fragrance diffuser, particularly for small spaces, and to the associated production process.

Diffusers of fragrances or essences for distributing fragrances in small spaces, such as for example cars, wardrobes, rooms, internal compartments of electrical household appliances or small areas are known. These diffusers, such as for example spray cans, can emanate into the surrounding environment the essence inside them directly.

These diffusers of the known type are not free from drawbacks, which include the fact of using metal containers which contain gas at high pressure which, if exposed to heat sources, can lead to the explosion of the container itself, becoming dangerous for nearby people. In addition to this, the expelled gases can be harmful to the environment and can alter the fragrance inside them, expelling essences that are different from the fragrance introduced initially.

Another drawback of these fragrance diffusers of the known type resides in the fact that they are potentially dangerous, since the atomization of the liquid fragrance into the environment, performed by automatic diffusers or unintentionally by the user, might contaminate foods, altering their edibility, or sensitive parts of the body, such as the eyes, causing burning and partial clouding of sight.

In addition to these diffusers of the known type, fragrance diffusers are known which indirectly, by using absorbent elements soaked in the essence, emanate said essence toward the surrounding environment.

These diffusers are composed of a hermetically sealed container, which accommodates internally a chamber for containing the essence and a membrane or other absorbent device which is at least partially immersed in said essence. Opening the container allows diffusing, by means of the membrane, the fragrance into the environment. Moreover, there can be an expansion seat, connected to the containment chamber, which accommodates part of the membrane. The diffusion of the essence toward the environment is furthermore correlated to the opening cross-section of the container; said opening must be performed by means of adapted devices or dedicated accommodation seats in which there are piercing elements which provide a sufficient opening in the container so as to diffuse uniformly, for a given period of time, the fragrance.

Further fragrance diffusers of the known type use an additional element that supports the volatilization of the essence, such as for example electric resistance heaters, small fans or other devices that require an external power supply in order to dispense the fragrance correctly in the environment.

These diffusers of the known type are not free from drawbacks, which include the fact that the membrane, which draws by capillary action the essence from the containment chamber, allows the passage of small molecules, preventing the diffusion of fragrances composed of molecules that are larger than the preceding ones.

Another drawback of these fragrance diffusers of the known type resides in the fact that they require a receptacle for the diffusion of the fragrance, since manual opening of the container might produce an opening that is larger than intended, altering the uniformity of the diffusion of the fragrance, and since it might also cause the escape of the fragrance in the liquid phase. Moreover, the receptacle, in addition to being an additional cost factor, allows the accommodation of a limited number of diffusers, reducing significantly the variety of fragrance diffusers that can be used.

A further drawback of these known types of fragrance diffusers resides in the fact that they cause a possible escape of the fragrance in the liquid phase when the container is opened. Fragrance diffusers comprising a membrane that is interposed between the fragrance containment chamber and the opening film in fact allow the passage of the liquid from the containment chamber to the interspace formed between the opening film and the membrane, and said liquid, during the opening of the device, i.e., during the elimination of the opening film, flows out in the liquid phase.

US 2011/180621 A1 discloses a fragrance dispenser comprising a main body which forms a containment chamber and an expansion seat, a membrane which faces the external environment and is proximate to the expansion seat, a passage channel interposed between the chamber and the seat, and the perimetric walls of the main body are made of deformable material.

US 4 998 671 A discloses the use of a barrier element to control fluid flow between a containment chamber and a dispensing chamber comprising a fluid diffusion portion. The fluid control concerns fluid direction and mixing and not fluid flow stoppage.

The aim of the present invention is to provide a fragrance diffuser, particularly for small spaces, that obviates the drawbacks and overcomes the limitations of the background art, allowing the dispensing of the fragrance without using containers at high internal pressure.

Within the scope of this aim, an object of the present invention is to provide a fragrance diffuser that allows the diffusion of fragrances composed of molecules that are larger than the molecules of the fragrances used for osmotic and permeable membranes.

A further object of the invention is to provide a fragrance diffuser that allows diffusion of the fragrance without atomizing the liquid directly in the surrounding environment.

Another object of the invention is to provide a fragrance diffuser that makes it possible to dispense the fragrance without requiring dedicated receptacles for the activation of the emanation.

A further object of the invention is to provide a fragrance diffuser that diffuses the essence without requiring external power supplies.

Another object of the invention is to provide a fragrance diffuser that avoids the escape of the fragrance in the liquid phase.

A further object of the invention is to provide a fragrance diffuser that is easy to provide and economically competitive if compared to the background art.

In accordance with the invention, there is provided a fragrance diffuser as defined in the appended claims 1-8.

A preferred method for manufacturing fragrance diffusers is defined in the appended claims 9-11.

Further characteristics and advantages of the present invention will become more apparent from the description of three preferred but not exclusive embodiments of a fragrance diffuser, particular for small spaces, illustrated by way of non-limiting example with the aid of the accompanying drawings, wherein:
Figure 1 is an exploded perspective view of a first embodiment of a fragrance diffuser, particularly for small spaces, according to the invention;
Figure 2 is a perspective view of the fragrance diffuser shown in Figure 1;
Figure 3 is a lateral elevation view of the fragrance diffuser shown in Figure 1;
Figure 4 is a sectional view of the fragrance diffuser shown in Figure 3, along the plane IV-IV;
Figures 5 and 6 are enlarged-scale views of a detail of the fragrance diffuser of Figure 4, respectively in the closed configuration and in the configuration for use;
Figure 7 is a perspective view of a second embodiment of the fragrance diffuser according to the invention;
Figure 8 is a lateral elevation view of the fragrance diffuser shown in Figure 7;
Figure 9 is a perspective view of a third embodiment of the fragrance diffuser according to the invention;
Figure 10 is a lateral elevation view of the fragrance diffuser shown in Figure 9.

With reference to the cited figures, the fragrance diffuser, particularly for small spaces, generally designated by the reference numeral 1, comprises a main body 8, which forms a containment chamber 3 and an expansion seat 4. The containment chamber 3 is conveniently segregated from the remaining part of the main body 8 for containing a fluid 100.

According to the invention, the fragrance diffuser 1 comprises a membrane 2 that faces the outside environment and is at least partly proximate to the expansion seat 4. In addition, the fragrance diffuser 1 has a closed configuration and a configuration for use; in the closed configuration, the fluid 100 is confined within the containment chamber 3, leaving the expansion seat 4 empty and the membrane 2 dry, whereas in the configuration for use the fluid 100, passing through the expansion seat 4, flows out of the containment chamber 3 toward the membrane 2, impregnating it and consequently evaporating into the surrounding environment.

Conveniently, the containment chamber 3 and the expansion seat 4 have open compartments formed by adapted contoured portions obtained by processes for deformation of the main body 8. In particular, the main body 8, which is initially sheetlike, has a front face 9 and a rear face 10 which are mutually opposite, and as a consequence of a deformation that can be obtained by means of known types of production processes, such as for example thermoforming processes or drawing processes, forms the containment chamber 3 and the expansion seat 4, which are completely closed on the front face 9, leaving respective portions open on the rear face 10. The main body 8 and its walls are made of a material that can be deformed elastically or plastically simply by means of the pressure that can be applied with one's fingers and can comprise a thermoplastic polymer adapted for molding and thermoforming, aluminum, polyethylene fibers or other materials depending on the specific requirements of application.

In contact with part of the rear face 10 there is a first face 11 of a sheetlike separator film 6, which has at least one opening 7 in the open portion of the expansion seat 4. The opening 7 can comprise a plurality of holes, a plurality of cuts or other slits which are connected between the two opposite faces of the separator film 6.

Conveniently, the separator film 6 adheres hermetically to the main body 8 along a first closed track 13 and a second closed track 14. Advantageously, this adhesion allows defining the complete closure of the containment chamber 3 and of the expansion seat 4, avoiding connections between them. Specifically, the containment chamber 3 has a closed isolated compartment that is formed by the first contoured portion of the main body 8 and by a portion of the separator film 6 that is connected to the former by means of the first closed track 13, whereas the expansion seat 4 has an open isolated compartment that is formed by the second contoured portion of the main body 8 and by another portion of the separator film 6 that is connected to the former by means of the second closed track 14, said compartment being open by virtue of the presence of the opening 7.

Preferably, the dimensions of the expansion seat 4 and of the containment chamber 3 are mutually correlated with a volume ratio comprised between 20% and 150%. Furthermore, the ideal quantity of fluid 100 inside the containment chamber 3 for a single-dose fragrance diffuser 1 varies between 1 and 20 milliliters.

The first closed track 13, which is proximate to the expansion seat 4, and the second closed track 14 are known technically as closure beads and can be obtained by means of a process for heat-sealing or adhesive bonding of the separator film 6 on the main body 8. Conveniently, the first closed track 13 has, on a limited portion 16 thereof, at least one passage channel 5, which once open connects the containment chamber 3 to the expansion seat 4. The passage channel 5 in fact is provided internally with at least one barrier partition 20, which in its closed configuration is active, closing the passage channel 5 and preventing the passage of the fluid 100 from the containment chamber 3 to the expansion seat 4, whereas in the configuration for use it is deactivated, opening the passage channel 5 and allowing the passage of the fluid 100.

In particular, the partition 20 has weakening lines designed to form fracture areas for the opening of the passage channel 5 and the consequent passage of the fluid 100. The weakening lines can comprise a smaller heat-sealed portion, a heat-sealed portion with a particular geometric shape that is different from the heat-sealing provided on the remaining portion of the first closed track 13, or a smaller amount of adhesive than the remaining portion of the first closed track 13, so that it can be divaricated after the increase in pressure within at least one of the containment chamber 3 and the expansion seat 4.

Finally, on the second face 12 of the separator film 6 there is the membrane 2, which is composed of a porous membrane 15. In particular, the porous membrane 15 is made of microporous material with an average passage porosity that can vary between 0.015 and 5 micrometers, preferably selected between 0.025 and 1 micrometer; the materials that compose it can be natural or synthetic, based on polyolefins or polyethylene, such as for example Teslin®, Solupor® or the like. Such a porous membrane 15 allows the passage of molecules of fragrances that are larger than the molecules of fragrance normally used in permeable and osmotic membranes.

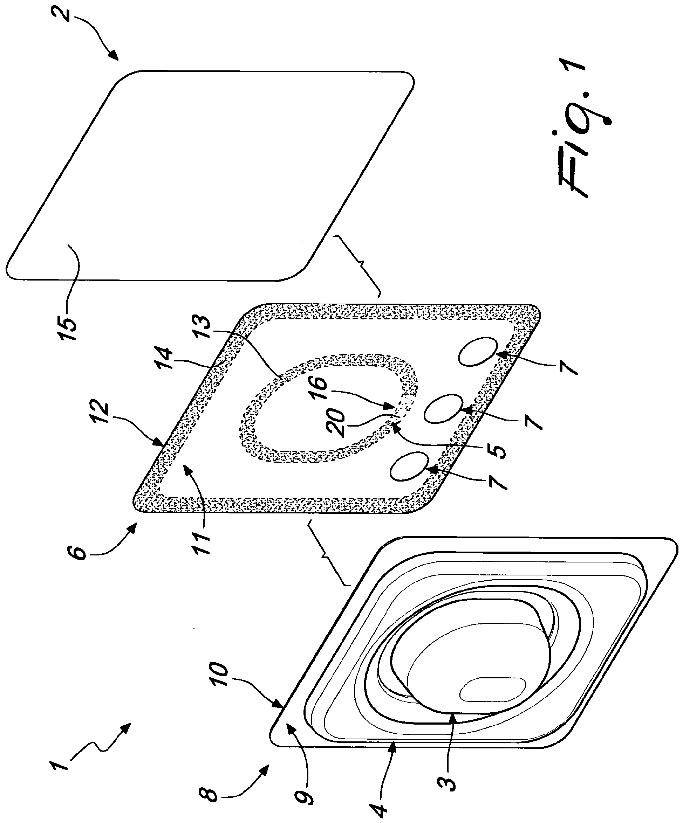

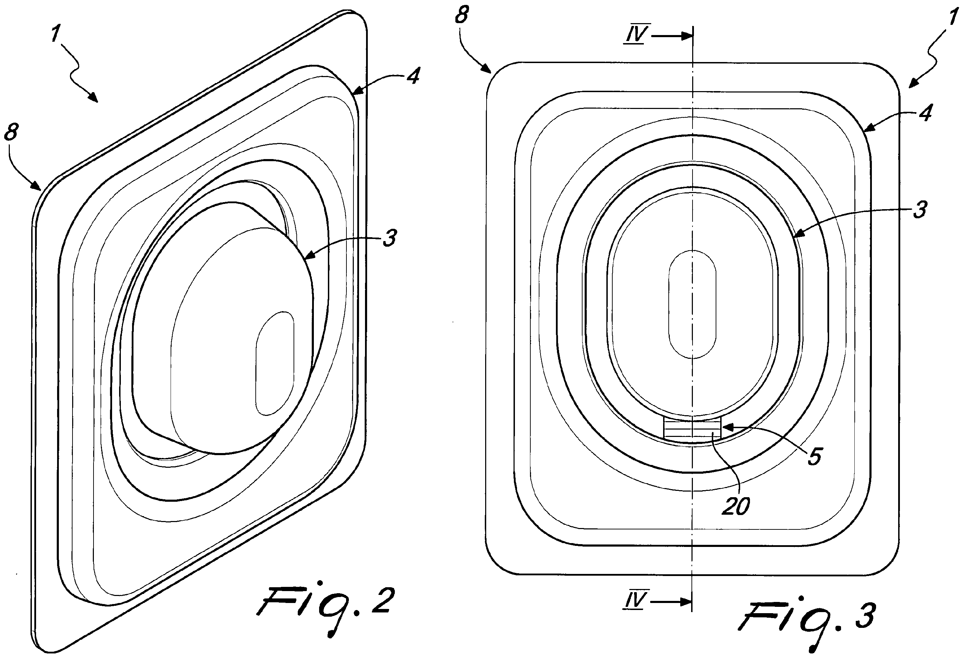

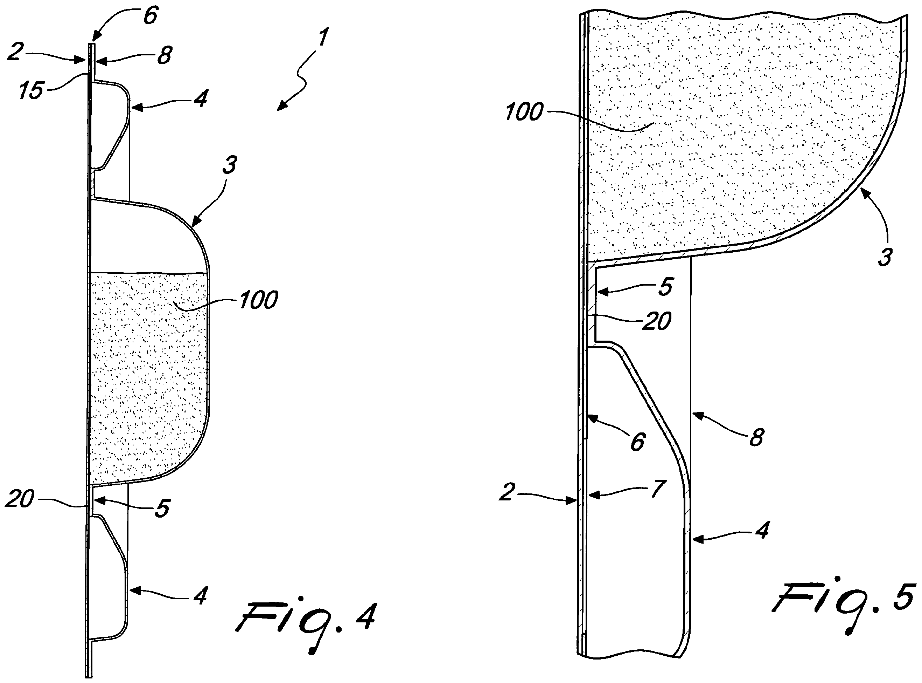

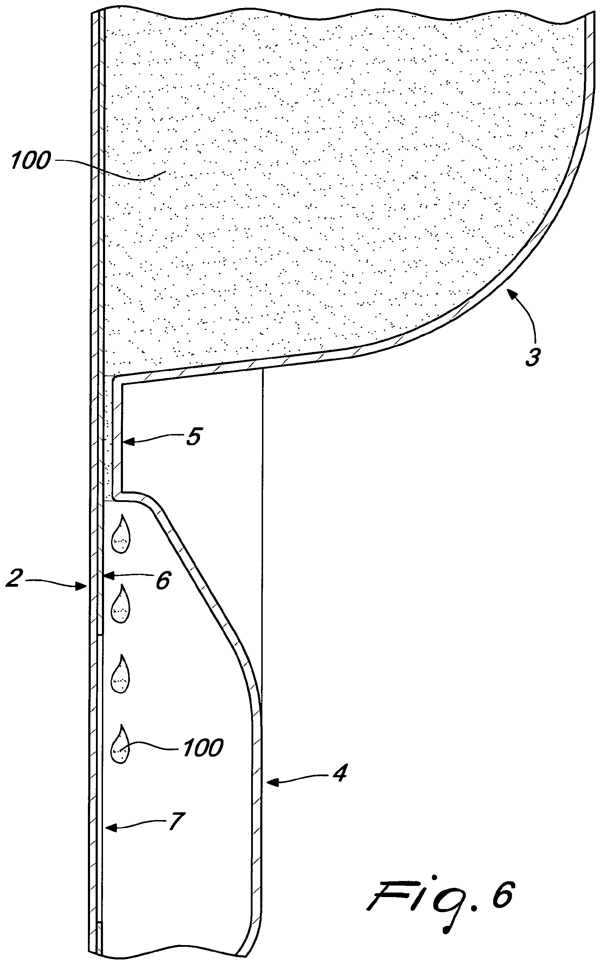

In a first embodiment, shown in Figures 1 to 6, the expansion seat 4 is arranged perimetrically on the main body 8, whereas the containment chamber 3 is arranged at the center of said body.

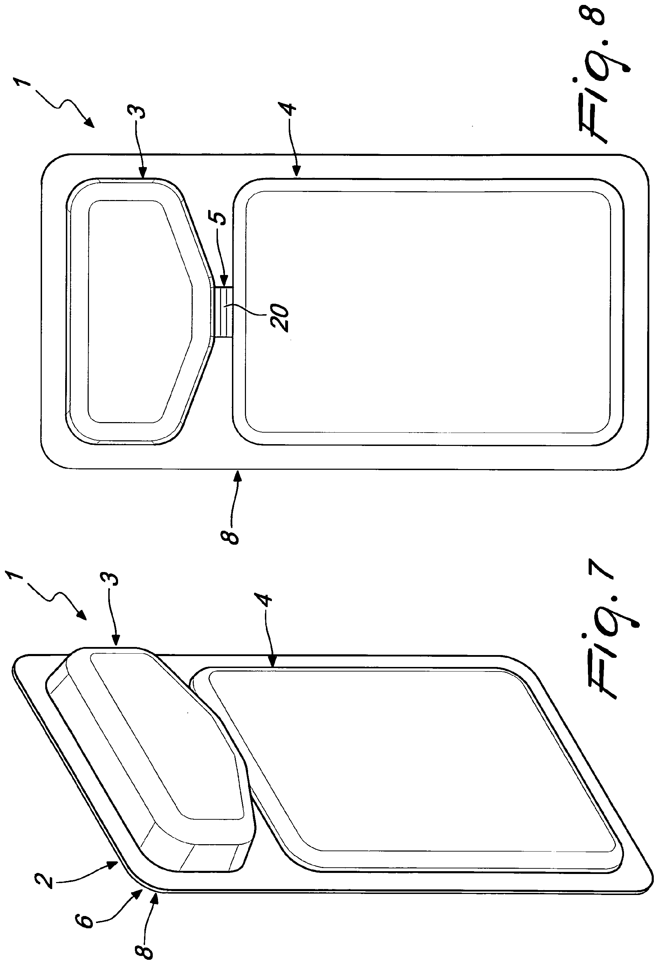

In a second embodiment, shown in Figures 7 and 8, the containment chamber 3 and the expansion seat 4 are arranged respectively on an upper portion and on a lower portion of the main body 8.

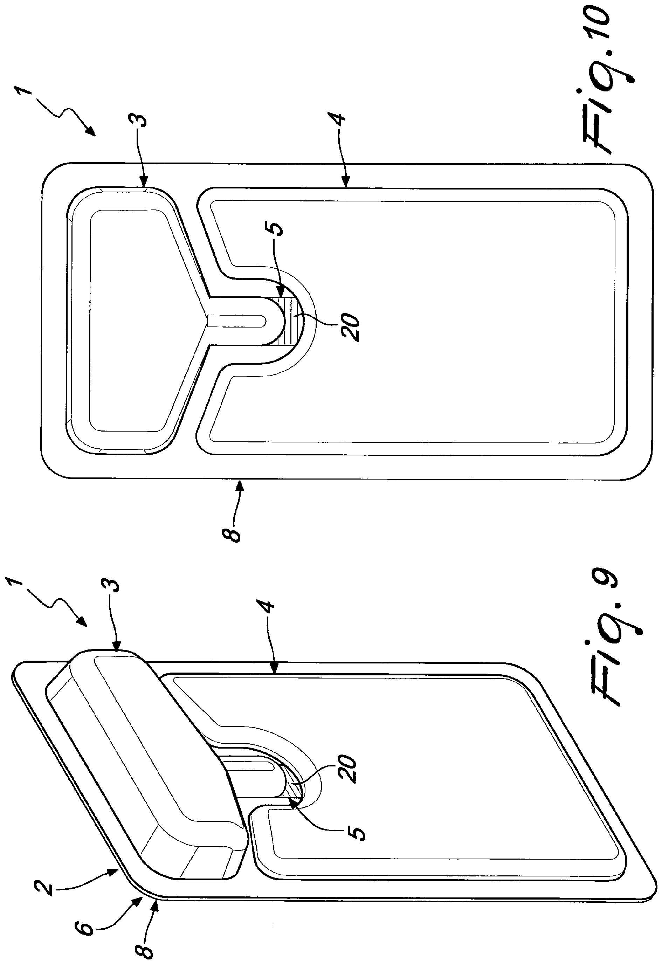

In a third embodiment, shown in Figures 9 and 10, the containment chamber 3 and expansion seat 4 are similar to the preceding embodiment, differing from it in a small extension of the containment chamber 3 proximate to the passage channel 5.

In another embodiment, not shown in the accompanying figures, the separator film 6 is delimited by the first closure track 13, leaving the expansion seat 4 in direct contact with the membrane 2. In this embodiment, therefore, the openings 7 are absent, since the fragrance, when it escapes from the containment chamber 3, is in direct contact with the membrane 2.

The fragrance diffuser 1, particularly for small spaces, which comprises a deformable main body 8, a separator film 6 and a membrane 3, can be provided by means of a process that comprises a first step of deformation of the main body 8 to form a containment chamber 3 and an expansion seat 4, which are respectively open onto a rear face 10 of the main body 8, and a second step of piercing or cutting the separator film 6 to obtain at least one through opening 7.

A third step of filling the containment chamber 3 with a fluid 100, normally in liquid form, in a quantity variable between 1 and 20 milliliters, depending on the capacity of the containment chamber 3, is performed. The third step can occur before the second step and vice versa, depending on the type of dedicated production.

Conveniently, a fourth step follows for juxtaposing a first face 11 of the separator film 6 on the rear face 10 so as to make the opening 7 match up at the open portion of the expansion seat 4, then a fifth step of adhesion of the separator film 6 on the rear face 10 along at least one first closed track 13 and a second closed track 14, which are mutually close, for the hermetic separation respectively of the container chamber 3 from the expansion seat 4. Finally, a sixth step of juxtaposition of the membrane 2 on a second face 12, which is opposite the first face 11, of the separator film 6 in contact with at least one opening 7, and a subsequent seventh step of adhesion of the membrane 3 on the separator film 6 to mutually couple the two elements are performed.

Advantageously, the fourth and sixth steps of juxtaposition and the respective adhesion steps can all be performed simultaneously as a consequence of the stratification of the various elements on each other or they can be performed simultaneously in pairs, i.e., the fourth step simultaneously with the fifth step and the sixth step simultaneously with the seventh step. It is noted that the two steps of adhesion can be performed by heat-sealing, adhesive bonding or other equivalent steps that allow the mutual adhesion of at least two elements. Moreover, the first step of deformation of the main body 8 occurs by means of a thermoforming or drawing process, depending on the material of which said body is made.

Conveniently, in the fifth heat-sealing step during the execution of the first closed track 13, a partition 20 is formed along a portion 16 of the latter, proximate to the expansion chamber 4, with weakening lines designed to form fracture areas that can be deformed under the action of external forces.

The operation of the fragrance diffuser 1, particularly for small spaces, is described hereinafter.

Initially, the fragrance diffuser 1 is in a closed configuration, in which the fluid 100 is confined within the containment chamber 3 and the partition 20 is active. Following a slight pressure applied to the external surface of one of the containment chamber 3 and the expansion seat 4, and particularly on the front face 9, an increase in pressure occurs inside them. The increase in pressure deforms the weakening lines of the partition 20, leading to the local separation of the main body 8 from the separator film 6, consequently deactivating the partition 20 and making the fragrance diffuser 1 pass from the closed configuration to the configuration for use. The fluid 100, which is present in the containment chamber 3, flows out by gravity or due to difference in pressure between the two compartments 3 and 4 toward the containment seat 4. The fluid 100 inside the expansion seat 4 is absorbed by the porous membrane 15 through the opening 7 and subsequently volatilizes into the surrounding environment.

In order to facilitate the activation of the partition 20 and the flow of the fluid 100 described above, it is possible to insert within the containment chamber 3 an inert gas at low pressure, such as for example nitrogen, in order to allow with minimal external pressure activation of the channel and a stronger outflow toward the expansion seat 4.

In an embodiment not shown in the accompanying figures, the partition 20, following its deactivation, can be reactivated, preventing the passage of the fluid 100 between the compartments 3 and 4. The partition 20 can be deactivated again following a subsequent external pressure on the containment chamber 3, returning the fragrance diffuser 1 to the configuration for use.

In practice it has been found that the fragrance diffuser, particular for small spaces, according to the present invention achieves the intended aim and objects, since it allows to dispense the fragrance into the environment without using high-pressure containers.

Another advantage of the fragrance diffuser according to the invention resides in the fact that it can diffuse fragrances composed of molecules that are larger than fragrances of the known type, by virtue of the presence of the membrane made of microporous material.

A further advantage of the fragrance diffuser according to the invention resides in the fact that it can diffuse the fragrance without atomizing it directly into the surrounding environment, since its emanation occurs by vaporization of the liquid on the membrane.

Another advantage of the fragrance diffuser according to the invention resides in the fact that it can deliver the essence without the aid of additional devices or dedicated receptacles, reducing the purchase cost significantly.

A further advantage of the fragrance diffuser according to the invention resides in the fact that it can deliver the essence without requiring external power supplies, such as for example electric or thermal supplies, allowing uniform dispensing of the fragrance even in environments lacking such supplies. The possibility is not excluded of using electrical devices that increase the intensity of fragrance release into the environment depending on the requirements of the user.

Another advantage of the fragrance diffuser according to the invention resides in the fact that it avoids the escape of the fragrance in the liquid phase, since said fragrance, segregated hermetically in the containment chamber, makes contact with the membrane after the user has opened the passage channel and then comes in contact with the air of the surrounding environment.

The fragrance diffuser particularly for small spaces thus conceived is susceptible of numerous modifications and variations, all of which are within the scope of the inventive concept.

All the details may further be replaced with other technically equivalent elements.

In practice, the materials used, as long as they are compatible with the specific use, as well as the contingent shapes and dimensions, may be any according to the requirements.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A fragrance diffuser (1), particularly for small spaces, comprising a main body (8) which forms a containment chamber (3) and an expansion seat (4), said containment chamber (3) being segregated from the remaining part of said main body (8) for containing a fluid (100), the diffuser (1) further comprising a membrane (2) which faces the external environment and is at least partially proximate to said expansion seat (4), and at least one passage channel (5), which is interposed between said containment chamber (3) and said expansion seat (4), provided internally with at least one barrier partition (20), said fragrance diffuser (1) having a closed configuration and a configuration for use, in said closed configuration said partition (20) being active to stop the passage of said fluid (100) from said containment chamber (3) to said expansion seat (4), in said configuration for use said partition (20) being inactive for the passage of said fluid (100) from said containment chamber (3) to said membrane (2), passing through said expansion seat (4), said main body (8) being provided with perimetric walls made of a deformable material.

2. The fragrance diffuser (1) according to claim 1, **characterized in that** it comprises a separator film (6) which is interposed between said main body (8) and said membrane (2), said separator film (6) being proximate at least to said containment chamber (3).

3. The fragrance diffuser (1) according to claim 2, **characterized in that** said separator film (6), which is proximate to said containment chamber (3) and to said expansion seat (4), has at least one opening (7) for the passage of said fluid (100) from said expansion seat (4) to said membrane (2) in said configuration for use.

4. The fragrance diffuser (1) according to claim 3, **characterized in that** said containment chamber (3) and said expansion seat (4) have respective closed compartments which are formed by adapted contoured portions of said main body (8) in combination with said separator film (6), said separator film (6) adhering to part of a rear face (10) of said main body (8) along at least one first closed track (13) and one second closed track (14) for the hermetic separation respectively of said containment chamber (3) from said expansion seat (4), said rear face (10) being opposite to a front face (9) that has said convex contoured portions.

5. The fragrance diffuser (1) according to one or more of the preceding claims 2-4, **characterized in that** said separator film (6) adheres to said membrane (2) with a second face (12) of its own, which is opposite to a first face (11) in contact with said main body (8).

6. The fragrance diffuser (1) according to one or more of the preceding claims 4-5, **characterized in that** said first closed track (13) has, on a portion (16) thereof proximate to said expansion seat (4), said passage channel (5).

7. The fragrance diffuser (1) according to one or more of the preceding claims, **characterized in that** said partition (20) has weakening lines which are designed to form fracture areas, said partition (20) being able to be activated by means of an internal pressure variation of at least one of said containment chamber (3) and said expansion seat (4).

8. The fragrance diffuser (1) according to one or more of the preceding claims, **characterized in that** said membrane (2) is a porous membrane (15) made of at least one microporous material with an average passage porosity which is substantially variable in the range of 0.015-5 micrometers.

9. A method for manufacturing fragrance diffusers (1), comprising a deformable main body (8), a separator film (6) and a membrane (2), **characterized in that** it comprises:
- a first step of deformation of said main body (8) in order to form a containment chamber (3) and an expansion seat (4) which are open respectively on a rear face (10) of said main body (8);
- a second step of piercing said separator film (6) in order to obtain at least one through opening (7);
- a third step of filling said containment chamber (3) with a fluid (100);
- a fourth step of juxtaposing a first face (11) of said separator film (6) on said rear face (10) so as to make said at least one opening (7) match up at the open portion of said expansion seat (4);
- a fifth step of adhesion of said separator film (6) on said rear face (10) along at least one first closed track (13) and one second closed track (14) for the hermetic separation respectively of said containment chamber (3) from said expansion seat (4);
- a sixth step of juxtaposition of said membrane (2) on a second face (12), which is opposite said first face (11), of said separator film (6) in contact with said at least one opening (7);
- a seventh step of adhesion of said membrane (2) to said separator film (6).

10. The method according to the preceding claim, **characterized in that** at least two of said fourth adhesion step, said fifth heat-sealing step, said sixth adhesion step, said seventh heat-sealing step are simultaneous.

11. The method according to claim 9, **characterized in that** said first closed heat-sealing track (13) comprises, in a portion (16) thereof, proximate to said expansion seat (4), a partition (20) which comprises weakening lines which are designed to form fracture areas, which can be deformed under the action of external forces, for the activation of said partition (20) and the consequent passage of said liquid from said containment chamber (3) to said expansion seat (4) through a passage channel (5).

## Patentansprüche

1. Ein Duftstoffdiffusor (1), insbesondere für kleine Räume, der einen Hauptkörper (8) umfasst, welcher eine Aufnahmekammer (3) und einen Expansionssitz (4) bildet, wobei die Aufnahmekammer (3) vom restlichen Teil des Hauptkörpers (8) getrennt ist, um ein Fluid (100) zu enthalten, wobei der Diffusor (1) weiter eine Membran (2) umfasst, die der äußeren Umgebung zugewandt ist und sich zumindest teilweise nahe dem Expansionssitz (4) befindet, und mindestens einen Durchgangskanal (5), der zwischen der Aufnahmekammer (3) und dem Expansionssitz (4) angeordnet ist, innen mit mindestens einer Trennwand (20) ausgestattet, wobei der Duftstoffdiffusor (1) eine geschlossene Anordnung und eine Gebrauchsanordnung hat, wobei die Trennwand (20) in der geschlossenen Anordnung aktiv ist, um den Übergang des Fluids (100) von der Aufnahmekammer (3) in den Expansionssitz (4) zu stoppen, und wobei in der Gebrauchsanordnung die Trennwand (20) inaktiv ist zum Zwecke des Übergangs des Fluids (100) von der Aufnahmekammer (3) zu der Membran (2), durch den Expansionssitz (4) dringend, wobei der Hauptkörper (8) mit Umfangswänden ausgestattet ist, die aus einem verformbaren Material bestehen.

2. Der Duftstoffdiffusor (1) gemäß Anspruch 1, **dadurch gekennzeichnet, dass** er einen Trennfilm (6) umfasst, der zwischen dem Hauptkörper (8) und der Membran (2) angeordnet ist, wobei der Trennfilm (6) sich mindestens nahe der Aufnahmekammer (3) befindet.

3. Der Duftstoffdiffusor (1) gemäß Anspruch 2, **dadurch gekennzeichnet, dass** der Trennfilm (6), der sich nahe der Aufnahmekammer (3) und dem Expansionssitz (4) befindet, mindestens eine Öffnung (7) für den Übergang des Fluids (100) von dem Expansionssitz (4) zu der Membran (2) in der Gebrauchsanordnung hat.

4. Der Duftstoffdiffusor (1) gemäß Anspruch 3, **dadurch gekennzeichnet, dass** die Aufnahmekammer (3) und der Expansionssitz (4) entsprechende geschlossene Abteilungen haben, die von geeigneten konturierten Abschnitten des Hauptkörpers (8) in Verbindung mit dem Trennfilm (6) gebildet werden, wobei der Trennfilm (6) an einem Teil einer hinteren Fläche (10) des Hauptkörpers (8) entlang mindestens einer ersten geschlossenen Bahn (13) und einer zweiten geschlossenen Bahn (14) haftet, zur entsprechenden hermetischen Trennung der Aufnahmekammer (3) von dem Expansionssitz (4), wobei die hintere Fläche (10) einer vorderen Fläche (9) gegenüberliegt, die die konvexe konturierte Abschnitte hat.

5. Der Duftstoffdiffusor (1) gemäß einem oder mehreren der obigen Ansprüche 2-4, **dadurch gekennzeichnet, dass** der Trennfilm (6) an der Membran (2) mit einer zweiten eigenen Fläche (12) haftet, die einer ersten Fläche (11), die in Kontakt mit dem Hauptkörper (8) ist, gegenüberliegt.

6. Der Duftstoffdiffusor (1) gemäß einem oder mehreren der obigen Ansprüche 4-5, **dadurch gekennzeichnet, dass** die erste geschlossene Bahn (13) in einem Abschnitt (16) derselben nahe dem Expansionssitz (4) den Durchgangskanal (5) hat.

7. Der Duftstoffdiffusor (1) gemäß einem oder mehreren der obigen Ansprüche, **dadurch gekennzeichnet, dass** die Trennwand (20) Schwächungslinien hat, die konstruiert sind, um Bruchzonen zu bilden, wobei die Trennwand (20) mit Hilfe einer inneren Druckschwankung der Aufnahmekammer (3) und/oder des Expansionssitzes (4) aktiviert werden kann.

8. Der Duftstoffdiffusor (1) gemäß einem oder mehreren der obigen Ansprüche, **dadurch gekennzeichnet, dass** die Membran (2) eine poröse Membran (15) ist, hergestellt aus mindestens einem mikroporösen Material mit einer durchschnittlichen Durchgangsporosität, die im Wesentlichen im Bereich von 0,015 - 5 Mikrometern variabel ist.

9. Ein Verfahren zur Herstellung von Duftstoffdiffusoren (1), die einen verformbaren Hauptkörper (8), einen Trennfilm (6) und eine Membran (2) umfassen, **dadurch gekennzeichnet, dass** es Folgendes umfasst:
- einen ersten Schritt des Verformens des Hauptkörpers (8), um eine Aufnahmekammer (3) und einen Expansionssitz (4) zu bilden, die jeweils auf einer Rückseite (10) des Hauptkörpers (8) offen sind;
- einen zweiten Schritt des Durchbohrens des Trennfilms (6), um mindestens eine Durchgangsöffnung (7) zu erhalten;
- einen dritten Schritt des Füllens der Aufnahmekammer (3) mit einem Fluid (100);
- einen vierten Schritt des Anordnens einer ersten Fläche (11) des Trennfilms (6) und der Rückseite (10) nebeneinander, um die mindestens eine Öffnung (7) mit dem offenen Abschnitt des Expansionssitzes (4) fluchten zu lassen;
- einen fünften Schritt der Adhäsion des Trennfilms (6) an der Rückseite (10) entlang mindestens einer ersten geschlossenen Bahn (13) und einer zweiten geschlossenen Bahn (14) zum Zwecke der hermetischen Trennung der Aufnahmekammer (3) von dem Expansionssitz (4);
- einen sechsten Schritt des nebeneinander Anordnens der Membran (2) und einer zweiten Fläche (12), die gegenüber der ersten Fläche (11) liegt, des Trennfilms (6), der in Kontakt mit der mindestens einen Öffnung (7) ist;
- einen siebten Schritt der Adhäsion der Membran (2) an dem Trennfilm (6).

10. Das Verfahren gemäß dem obigen Anspruch, **dadurch gekennzeichnet, dass** mindestens zwei Schritte des vierten Adhäsionsschritts, des fünften Wärmeversiegelungsschritts, des sechsten Adhäsionsschritts, und des siebten Wärmeversiegelungsschritts gleichzeitig stattfinden.

11. Das Verfahren gemäß Anspruch 9, **dadurch gekennzeichnet, dass** die erste geschlossene Wärmeversiegelungsbahn (13) in einem Abschnitt (16) derselben, nahe dem Expansionssitz (4), eine Trennwand (20) umfasst, die Schwächungslinien umfasst, welche konstruiert sind, um Bruchzonen zu bilden, die unter der Wirkung äußerer Kräfte verformt werden können, zum Zwecke der Aktivierung der Trennwand (20) und des sich daraus ergebenden Übergangs der Flüssigkeit von der Aufnahmekammer (3) in den Expansionssitz (4) durch einen Durchgangskanal (5).

## Revendications

1. Diffuseur de parfum (1), notamment pour petits espaces, qui comprend un corps principal (8) qui forme une chambre de confinement (3) et un logement d'expansion (4), ladite chambre de confinement (3) étant séparée de la partie restante dudit corps principal (8) pour contenir un fluide (100), le diffuseur (1) comprenant, en plus, une membrane, qui est tournée vers l'environnement externe et qui est au moins partiellement à proximité du logement d'expansion (4), et au moins un canal de passage (5) qui est interposé entre ladite chambre de confinement (3) et ledit logement d'expansion (4), doté, à l'intérieur, d'au moins une barrière de séparation (20), ledit diffuseur de parfum (1) ayant une configuration fermée et une configuration d'utilisation, dans ladite configuration fermée, ladite séparation (20) est active pour stopper le passage dudit fluide (100) depuis la chambre de confinement (3) vers ledit logement d'expansion (4), dans ladite configuration d'utilisation, ladite séparation (20) est inactive pour le passage dudit fluide (100) depuis ladite chambre de confinement (3) vers ladite membrane (2) en passant à travers ledit logement d'expansion (4), ledit corps principal (8) étant pourvu de parois périmétriques réalisées en matière déformable.

2. Diffuseur de parfum (1) suivant la revendication 1, **caractérisé en ce qu'**il comprend un film séparateur (6) qui est interposé entre ledit corps principal (8) et ladite membrane (2), ledit film séparateur (6) étant situé à proximité d'au moins ladite chambre de confinement (3).

3. Diffuseur de parfum (1) suivant la revendication 2, **caractérisé en ce que** ledit film séparateur (6), qui est situé à proximité de ladite chambre de confinement (3) et dudit logement d'expansion (4), présente au moins une ouverture (7) pour le passage dudit fluide (100) depuis ledit logement d'expansion (4) vers ladite membrane (2) dans ladite configuration d'utilisation.

4. Diffuseur de parfum (1) suivant la revendication 3, **caractérisé en ce que** ladite chambre de confinement (3) et ledit logement d'expansion (4) ont des compartiments respectivement fermés qui sont formés par des portions incurvées adaptées dudit corps principal (8) en combinaison avec ledit film séparateur (6), ledit film séparateur (6) adhérant à une partie de la face arrière (10) dudit corps principal (8), le long d'au moins un premier parcours fermé (13) et d'un second parcours fermé (14) pour la séparation hermétique respective de ladite chambre de confinement (3) par rapport audit logement d'expansion (4), ladite face arrière (10) étant opposée à une face avant (9) qui présente lesdites portions incurvées convexes.

5. Diffuseur de parfum (1) suivant une ou plusieurs des revendications précédentes 2 à 4, **caractérisé en ce que** ledit film séparateur (6) adhère à ladite membrane (2) avec sa seconde face (12) qui est opposée à une première face (11) en contact avec ledit corps principal (8).

6. Diffuseur de parfum (1) suivant une ou plusieurs des revendications précédentes 4 à 5, **caractérisé en ce que** ledit premier parcours fermé (13) présente sur une portion à proximité dudit logement d'expansion (4) ledit canal de passage (5).

7. Diffuseur de parfum (1) suivant une ou plusieurs des revendications précédentes, **caractérisé en ce que** ladite séparation (20) présente des lignes d'affaiblissement qui sont conçues pour former des zones de rupture, ladite séparation (20) pouvant être activée au moyen d'une variation de pression interne d'au moins un parmi ladite chambre de confinement (3) et ledit logement d'expansion (4).

8. Diffuseur de parfum (1) suivant une ou plusieurs des revendications précédentes, **caractérisé en ce que** ladite membrane (2) est une membrane poreuse (15) réalisée en au moins une matière microporeuse avec une porosité de passage moyenne qui varie essentiellement dans une plage allant de 0,015 à 5 micromètres.

9. Procédé de fabrication de diffuseurs de parfum (1) comprenant un corps principal déformable (8), un film séparateur (6) et une membrane (2), **caractérisé en ce qu'**il comprend :
- une première étape de déformation dudit corps principal (8) de façon à former une chambre de confinement (3) et un logement d'expansion (4) qui sont ouverts respectivement sur une face arrière (10) dudit corps principal (8),
- une seconde étape de perçage dudit film séparateur (6) de façon à obtenir au moins une ouverture traversante (7),
- une troisième étape de remplissage de ladite chambre de confinement (3) avec un fluide (100),
- une quatrième étape de juxtaposition d'une première face (11) dudit film séparateur (6) sur ladite face arrière (10) de façon à assurer qu'au moins une ouverture (7) corresponde à la portion ouverte dudit logement d'expansion (4),
- une cinquième étape d'adhésion dudit film séparateur (6) sur ladite face arrière (10), le long d'au moins un premier parcours fermé (13) et d'un second parcours fermé (14), pour la séparation hermétique respective de ladite chambre de confinement (3) par rapport audit logement d'expansion (4),
- une sixième étape de juxtaposition de ladite membrane (2) sur une seconde face (12), opposée à ladite première face (11), dudit film séparateur (6) en contact avec ladite au moins une ouverture (7),
- une septième étape d'adhésion de ladite membrane (2) sur ledit film séparateur (6).

10. Procédé suivant la revendication précédente, **caractérisé en ce qu'**au moins deux parmi ladite quatrième étape d'adhésion, ladite cinquième étape de thermoscellage, ladite sixième étape d'adhésion et ladite septième étape de thermoscellage ont lieu simultanément.

11. Procédé suivant la revendication 9, **caractérisé en ce que** ledit premier parcours fermé thermoscellé (13) comprend, dans une portion (16) à proximité dudit logement d'expansion (4), une séparation (20) comprenant des lignes d'affaiblissement conçues pour former des zones de rupture qui peuvent être déformées sous l'action de forces externes pour l'activation de ladite séparation (20) et le passage, qui en résulte, dudit liquide depuis ladite chambre de confinement (3) vers ledit logement d'expansion (4) à travers un canal de passage (5).
